**Europäisches Patentamt**

**European Patent Office**

**Office européen des brevets**

(19)

(11)    **EP 1 262 563 A2**

(12)    **EUROPEAN PATENT APPLICATION**

(43) Date of publication:
    **04.12.2002  Bulletin 2002/49**

(51) Int Cl.[7]: **C12Q 1/68**

(21) Application number: **02011709.9**

(22) Date of filing: **24.05.2002**

(84) Designated Contracting States:
    **AT BE CH CY DE DK ES FI FR GB GR IE IT LI LU
    MC NL PT SE TR**
    Designated Extension States:
    **AL LT LV MK RO SI**

(30) Priority: **31.05.2001  JP 2001164846**

(71) Applicant: **TAKARA BIO INC.
    Otsu-shi, Shiga (JP)**

(72) Inventors:
    • **Yamashita, Shusaku
      Otsu-shi, Shiga (JP)**

    • **Koyama, Nobuto
      Uji-shi, Kyoto (JP)**
    • **Mineno, Junichi
      Uji-shi, Kyoto (JP)**
    • **Kato, Ikunoshin
      Uji-shi, Kyoto (JP)**

(74) Representative: **Grund, Martin, Dr. et al
    Dr. Volker Vossius
    Patentanwaltskanzlei - Rechtsanwaltskanzlei
    Geibelstrasse 6
    81679 München (DE)**

(54)    **Method for immobilizing DNA on a surface**

(57)    A method for immobilizing a DNA upon preparation of a DNA microbead array, the method comprising forming a covalent bond between a DNA to be immobilized and an anti-tag DNA on a microbead using a DNA polymerase and a DNA ligase, wherein the DNA polymerase and the DNA ligase are reacted at the same time.

**EP 1 262 563 A2**

**Description**

BACKGROUND OF THE INVENTION

Field of the Invention

The present invention relates to a method for forming a covalent bond between an anti-tag sequence attached to a microbead and a DNA to be immobilized onto the microbead in the DNA microbead array technique, as well as a DNA microbead array prepared using said method.

Description of Related Art

The DNA microbead array technique is described in detail in Brenner et al., Proc. Natl. Acad. Sci. USA, 97: 1665-1670, 2000 (hereinafter referred to as Literature 1). In the DNA microbead array technique, hybridization of a single-stranded tag attached to a DNA to be immobilized onto a microbead (hereinafter referred to as a target DNA) with an anti-tag attached to the bead makes a single target DNA correspond to a single microbead. A microbead to which a target DNA is bound as a result of the hybridization is selected using a cell sorter. It is then subjected to various enzymatic and chemical treatments to covalently attach the target DNA to the anti-tag sequence. An adaptor is added to the terminus of the target DNA distal to the microbead. The target DNA is then converted into single-stranded DNAs for use in competitive hybridization.

Brenner et al. sequentially carry out the enzymatic and chemical reactions in the Literature 1 as follows: 1) repair of gaps using T4 DNA polymerase; 2) degradation of T4 DNA polymerase using Pronase; 3) ligation of nicks using T4 DNA ligase; 4) degradation of T4 DNA ligase using Pronase; 5) removal of DNA fragments containing fluorescent groups covalently attached to microbeads using DpnII; 6) degradation of DpnII using Pronase; 7) removal of 5'-phosphate groups using a phosphatase; 8) ligation of adaptors bearing fluorescent groups using T4 DNA ligase; 9) degradation of the phosphatase and T4 DNA ligase using Pronase; 10) 5'-phosphorylation of nicked sites using T4 polynucleotide kinase and ligation of nicks using T4 DNA ligase; 11) degradation of T4 polynucleotide kinase and T4 DNA ligase using Pronase; and 12) conversion of target DNAs into single-stranded DNAs using NaOH.

Brenner et al. sequentially carry out the reactions as described below following the step 5 (the DpnII reaction) in another literature (Nature Biotechnology, 18:630-634, 2000; hereinafter referred to as Literature 2): 6') modification of DpnII-digested termini using T4 DNA polymerase in the presence of dGTP; and 7') ligation of adaptors using T4 DNA ligase. In this case, target DNAs are not converted into single-stranded DNAs because microbeads having double-stranded DNAs being immobilized are used in the subsequent procedure of determining nucleotide sequences of the target DNAs.

As described above, the methods of Brenner et al. require four steps of enzymatic reactions using T4 DNA polymerase, Pronase (a composition containing plural proteases), T4 DNA ligase and Pronase in the process of formation of covalent bonds between anti-tag sequences on microbeads and target DNAs. This series of reactions makes the operation complicated. In addition, the reaction efficiency is about 60%, not rarely below 30%. Furthermore, Pronase may have serious harmful influences on the subsequent enzymatic reactions unless it is completely washed away after the Pronase reaction. For the reasons as described above, a convenient and efficient method for forming covalent bonds between anti-tag sequences on microbeads and target DNAs has been desired.

SUMMARY OF THE INVENTION

The main object of the present invention is to provide means of increasing the reaction efficiency, simplifying the operation, and decreasing the risk of reduction in reaction efficiencies of subsequent steps due to incomplete removal of a protease in the process of formation of covalent bonds between anti-tag sequences attached to microbeads and target DNAs upon preparation of DNA microbead arrays.

As a result of intensive studies, the present inventors have found that the operation can be simplified by reacting a DNA polymerase (e.g., T4 DNA polymerase) and a DNA ligase (e.g., T4 DNA ligase) at the same time in the above-mentioned process. Furthermore, the present inventors have found that this method greatly increases the reaction efficiency. Thus, the present invention has been completed.

The present invention is outlined as follows. The first aspect of the present invention relates to a method for immobilizing a DNA upon preparation of a DNA microbead array, the method comprising forming a covalent bond between a DNA to be immobilized and an anti-tag DNA on a microbead using a DNA polymerase and a DNA ligase, wherein the DNA polymerase and the DNA ligase are reacted at the same time.

A DNA polymerase that substantially has a DNA synthesis activity as well as a 5'→3' exonuclease activity and/or a 3'→5' exonuclease activity can be preferably used as the DNA polymerase in the first aspect. Furthermore, although it is not intended to limit the present invention, it is preferably to use, for example, at least one selected from the group consisting of T4 DNA polymerase, Escherichia coli DNA polymerase I, the Klenow fragment of Escherichia coli DNA polymerase I, Taq DNA polymerase and Pfu DNA polymerase as the DNA polymerase in the first aspect.

Although it is not intended to limit the present invention, for example, at least one selected from the group consisting of T4 DNA ligase, Escherichia coli DNA ligase and Taq DNA ligase can be preferably used as the DNA ligase in the first aspect.

Although it is not intended to limit the present invention, the concentrations of the DNA polymerase and the DNA ligase used in the first aspect preferably range, for example, from 20 to 2,000 U/ml and from 200 to 50,000 U/ml, respectively.

The second aspect of the present invention relates to a DNA microbead array prepared using the method of the first aspect.

DETAILED DESCRIPTION OF THE INVENTION

The present invention is described in detail below.

As used herein, a DNA microbead array technique means the technique as described in the Literature 1 or 2 by Brenner et al., or a technique substantially equivalent thereto. Specifically, it is a technique for preparing a library of microbeads having target DNAs being immobilized by placing specific target DNAs on specific microbeads as a result of hybridization of anti-tag sequences attached to the microbeads with tag sequences attached to the target DNAs. In this technique, a single target DNA is immobilized on a single microbead.

There is no specific limitation concerning the material of the microbead. It may vary depending on the purpose. Examples of the materials include glass, low-crosslinked polystyrene, highly crosslinked polystyrene, glycidal methacrylate and magnetic materials. Furthermore, there is no specific limitation concerning the size of the microbead. For example, the size may be from 1 to 100 $\mu$m in diameter although it may vary depending on the purpose.

As used herein, a single-stranded tag means an oligonucleotide tag as disclosed in JP-A 10-507357 (corresponding to WO 96/12014; hereinafter referred to as Literature 3).

As used herein, an anti-tag means an oligonucleotide that is completely complementary to the single-stranded tag. A double-stranded product generated as a result of annealing of the single-stranded tag to the anti-tag is called a tag or a double-stranded tag.

The single-stranded tags, the anti-tags, the tags and the double-stranded tags may be oligonucleotides that are not covalently attached to other molecules. Alternatively, they may be covalently attached to other DNAs, microbeads, fluorescent materials, or other molecules. Furthermore, some or all of nucleotides may be replaced by modified nucleotides such as, without limitation, 5-methyl cytosine, 7-deaza guanine or 6-methyl adenine.

As used herein, a tag sequence means a sequence of an oligonucleotide used as the single-stranded tag or the anti-tag. Although there is no specific limitation concerning its structure, the tag sequence is exemplified by one containing plural subunits each consisting of an oligonucleotide of 3 to 6 bases. Although it is not intended to limit the present invention, as an example, the anti-tag sequences as described in the Literature 1 can be preferably used. Specifically, they are oligonucleotides of 32 bases each consisting of eight "words" connected each other. The "word" corresponds to the "subunit" in the Literature 3 and is selected from the group consisting of TTAC, AATC, TACT, ATCA, ACAT, TCTA, CTTT and CAAA. The anti-tag sequences consist of $8^8$ (= about 17 million) sequences. The single-stranded tags are oligonucleotides of 32 bases each being complementary to the anti-tags, and consist of about 17 million sequences like the anti-tags. Furthermore, the double-stranded tags are double-stranded oligonucleotides of 32 base pairs generated as a result of annealing of the single-stranded tags to the anti-tags each having a sequence completely complementary to one of the single-stranded tags. The double-stranded tags also consist of about 17 million sequences.

As used herein, a target DNA means a DNA immobilized or to be immobilized onto a microbead. There is no limitation concerning its origin. For example, it may be derived from an animal, a plant, an eukaryotic microorganism, a prokaryotic microorganism or a virus. Also, there is no limitation concerning the method for preparing the target DNA. A genomic DNA, a cDNA, a synthetic DNA, a PCR product, a restriction fragment therefrom, or a physical- or chemical-treatment product therefrom can be preferably used. In other words, any naturally occurring or artificially prepared DNA can be preferably used as the target DNA as long as it can be immobilized onto a microarray used according to the present invention.

As used herein, a DNA microbead array means a collection of microbeads having target DNAs being immobilized through covalent bonds, which is prepared using the DNA microbead array technique. The form of the collection is not specifically limited. For example, it may be a collection of microbeads having restriction fragments from cDNAs being immobilized, the cDNA being prepared from substantially all of the mRNAs expressed in HL-60 cells.

As used herein, a DNA to be immobilized refers to a double-stranded target DNA to which a single-stranded tag is covalently attached. A substance other than a DNA such as a fluorescent substance (e.g., 6-carboxyl fluorescein (FAM)), biotin or a hapten (e.g., digoxigenin) may be additionally attached thereto through a covalent bond. According to the method of the present invention, the density of DNAs to be immobilized onto a 5-μm microbead is preferably $10^3$ to $10^6$ molecules per microbead, more preferably $10^4$ to $10^5$ molecules per microbead.

There is no specific limitation concerning the DNA polymerase used according to the present invention as long as it has an activity of synthesizing a DNA that is complementary to a DNA as a template. One having only a DNA synthesis activity as well as one additionally having a 5'→3' exonuclease activity and/or a 3'→5' exonuclease activity may be preferably used. A naturally occurring or mutant DNA polymerase having the enzymatic activity (or activities) as described above may be preferably used. For example, mesophilic enzymes such as T4 DNA polymerase, Escherichia coli DNA polymerase I and the Klenow fragment of Escherichia coli DNA polymerase I, as well as DNA polymerases from thermophilic bacteria such as Taq (Thermus aquaticus) and Pfu (Pyrococcus furiosus) may be preferably used. For example, in case of T4 DNA polymerase, the concentrations of the DNA polymerase upon reaction used in the method of the present invention range, without limitation, preferably from 20 to 2,000 U/ml, more preferably from 100 to 500 U/ml according to the unit (U) value indication as described in New England Biolabs catalogue 2000-2001, pp.83.

Any DNA ligase that can ligate nicks in double-stranded DNAs may be used according to the present invention. For example, T4 DNA ligase, Escherichia coli DNA ligase and Taq (T. aquaticus) DNA ligase may be preferably used. For example, in case of T4 DNA ligase, the concentrations of the DNA ligase used in the method of the present invention range, without limitation, preferably from 200 to 50,000 U/ml, more preferably from 1,000 to 10,000 U/ml according to the unit (U) value indication as described in New England Biolabs catalogue 2000-2001, pp.94.

According to the method of the present invention, there is no specific limitation concerning the composition of the reaction mixture for allowing a DNA polymerase and a DNA ligase to act as long as both enzymes exhibit their activities in the mixture. For example, a buffer at about neutral pH containing $MgCl_2$, NaCl, DTT and Tween 20 can be preferably used. At least one (preferably all) of dATP, dGTP, dTTP and dCTP as a substrate (or substrates) for the DNA polymerase is (are) added to such a buffer. Some or all of these nucleotides may be nucleotides containing a modified base (e.g., 5-methyl dCTP, 7-deaza dGTP or 6-methyl dATP). In addition to the above, an AMP donor required for a DNA ligase reaction is added. The AMP donor may be appropriately selected depending on the type of the DNA ligase. In case of T4 DNA ligase, ATP can be preferably used, whereas nicotinamide adenine dinucleotide (NAD) can be preferably used for Escherichia coli DNA ligase.

There is no specific limitation concerning the time or the temperature for a reaction using a DNA polymerase or a DNA ligase in the method of the present invention. The time and the temperature vary depending on the type or the concentration of the DNA polymerase or the DNA ligase to be used, or the concentration of a target DNA as a substrate on a microbead, and may be appropriately determined. For example, if a reaction is carried out using T4 DNA polymerase at a concentration of 200 U/ml and T4 DNA ligase at a concentration of 4,000 U/ml in a 100-μl reaction mixture containing two million microbeads, the reaction time is preferably 10 minutes to 5 hours, more preferably 30 minutes to 2 hours, and the reaction temperature is preferably 0 to 37°C, more preferably 5 to 20°C.

There is no specific limitation concerning the protease used according to the present invention as long as it completely or partially degrade the DNA polymerase, the DNA ligase and DpnII to abolish their enzymatic activities. For example, Pronase, proteinase K, trypsin, chymotrypsin or the like may be preferably used. Pronase is particularly preferable according to the present invention. The concentration of the protease used in the method of the present invention may be appropriately determined depending on the type of the protease to be used. For example, in case of Pronase, the concentrations range preferably from 10 to 2,000 μg/ml, more preferably from 50 to 500 μg/ml. Furthermore, the reaction time and the reaction temperature may be appropriately determined depending the type of the protease to be used in the method of the present invention. For example, in case of Pronase, the reaction time is preferably 10 minutes to 5 hours, more preferably 30 minutes to 2 hours, and the reaction temperature is preferably 10 to 50°C, more preferably 20 to 40°C.

For example, a microbead to which a target DNA is bound as a result of hybridization, to which the DNA immobilization method of the present invention is applied, can be prepared as follows. One embodiment of the method of the present invention is described below. The DNA immobilization method of the present invention can also be applied to a microbead to which a target DNA is bound which is prepared by another method.

A PCR primer-binding sequence followed by an anti-tag sequence is first synthesized on a microbead according to the phosphoramidite method. The synthesis is carried out according to the "mix and divide" synthesis method. This is because it is necessary to obtain a collection of a large number of (for example, about 17 million) microbeads having anti-tags such that each anti-tag having a single sequence is synthesized on a single microbead.

An additional PCR primer-binding sequence is added to a portion of the microbeads. Double-stranded tags are amplified by a PCR using a combination of primers sandwiching the anti-tags. A mixture of the amplified double-stranded tags is ligated into a plasmid vector to obtain a mixture of about 17 million plasmids having the double-stranded tags being inserted (called a tag vector).

Preparation of a microbead array having cDNAs derived from animal cells being immobilized is described below as an example. First, cDNAs are synthesized using mRNAs prepared from animal cells as templates. Restriction fragments from the double-stranded cDNAs are inserted into the tag vector to prepare a cDNA library. The restriction fragments from the double-stranded cDNAs serve as target DNAs. Regions each containing a target DNA and a double-stranded tag are amplified by a PCR using the mixture of the plasmids as a template. A primer labeled with an appropriate labeling compound such as a fluorescent substance (e.g., 6-carboxyl fluorescein (FAM)) at the 5'-terminus may be used as a primer for the target DNA side. Examples of other labeling compounds that can be preferably used include Cy5, Cy3, biotin and digoxigenin. If a non-fluorescent labeling compound is used, a fluorescent substance can be attached to the target DNA by using a fluorescent compound having an affinity for the labeling compound, for example, fluorescence-labeled avidin in case of labeling with biotin.

Next, the primer sequence on the double-stranded tag side is removed from each of the PCR-amplified DNA fragments by treatment with a restriction enzyme. One of the two strands of each double-stranded tag is degraded by treatment with T4 DNA polymerase in the presence of dGTP to obtain a double-stranded cDNA to which a single-stranded tag is attached.

The double-stranded target DNAs to which single-stranded tags are attached and the collection of microbeads having anti-tags are mixed together and subjected to hybridization. After washing and removing nonspecifically adsorbed target DNAs, the hybridization mixture is subjected to a cell sorter to isolate microbeads emitting fluorescence as a result of binding of the target DNAs.

A covalent bond is formed as follows between a target DNA and an anti-tag on the thus obtained microbead to which the target DNA is bound as a result of hybridization. Specifically, the method of the present invention may comprise the following two steps: 1) repairing gaps and forming covalent bonds at the same time by simultaneously reacting T4 DNA polymerase and T4 DNA ligase in the presence of a mixture of four deoxynucleoside triphosphates as well as ATP; 2) degrading T4 DNA polymerase and T4 DNA ligase by treatment with a protease. Either of the deoxynucleoside triphosphates may be a modified nucleotide such as 5-methyl dCTP, 7-deaza dGTP or 6-methyl dATP.

The fluorescence intensity for microbeads without (Sample A) or with (Sample B) heating after completion of covalent bond formation reaction is measured using a flow cytometer. Then, the efficiency of covalent bond formation reaction can be calculated according to the following equation:

Efficiency of covalent bond formation reaction (%) = (Mean fluorescence intensity for Sample B) / (Mean fluorescence intensity for Sample A) x 100

There is no specific limitation concerning the heating conditions as long as the bond between the tag and the anti-tag as a result of hybridization is dissociated. For example, heating in 10 mM Tris-HCl buffer (pH 8.0) containing 1 mM EDTA and 0.01% Tween 20 may be carried out at 50°C to 100°C, preferably 60°C to 90°C, more preferably 70°C to 80°C.

As compared with the method of the Literature 1, the operation of the method of the present invention is more convenient because it comprises fewer steps. Furthermore, it can reduce the loss of microbeads during the operation. The efficiency of covalent bond formation reaction in the conventional method is usually about 60 to 70%, often below 30%. On the other hand, the efficiency of covalent bond formation reaction in the method of the present invention is usually 80% or more, preferably 90% or more. Thus, the reaction efficiency and the reproducibility of the method of the present invention are higher than those of the conventional method.

Microbeads having single-stranded target DNAs being immobilized may be prepared, for example, by carrying out the following reactions as described in the Literature 1 in order to use the target DNAs immobilized onto the microbeads for hybridization with nucleic acids contained in samples: 1) removal of DNA fragments containing fluorescent groups covalently attached to microbeads using DpnII; 2) degradation of DpnII using a protease; 3) modification of DpnII-digested termini using the large fragment of DNA polymerase I in the presence of dGTP; 4) degradation of the large fragment of DNA polymerase I using a protease; 5) ligation of adaptors using T4 DNA ligase; 6) degradation of T4 DNA ligase using a protease; and 7) conversion of target DNAs into single-stranded DNAs using NaOH.

The fluorescence intensity for microbeads after completion of the reaction for degrading T4 DNA ligase using a protease (e.g., Pronase) (Sample C) is measured using a flow cytometer. Then, the efficiency of adaptor ligation reaction can be calculated according to the following equation:

Efficiency of adaptor ligation reaction (%) = (Mean fluorescence intensity for Sample C) / (Mean fluorescence intensity for Sample A) x 100

The fluorescence intensity for microbeads after completing conversion of target DNAs into single-stranded DNAs using NaOH (Sample D) is measured using a flow cytometer. Then, the efficiency of conversion of target DNAs into single-stranded DNAs can be calculated according to the following equation:

Efficiency of conversion of target DNAs into single-stranded DNAs (%) = {1 - (Mean fluorescence intensity for Sample D) / (Mean fluorescence intensity for Sample C)} x 100

Both the efficiency of adaptor ligation reaction and the efficiency of conversion of target DNAs into single-stranded DNAs in the method as described in the Literature 1 are usually about 90%. The present inventors have found that the efficiency of adaptor ligation reaction and the efficiency of conversion of target DNAs into single-stranded DNAs in the method of the present invention are equivalent to those of the method of the Literature 1 even if the reaction for degrading the large fragment of DNA polymerase I using Pronase is omitted from the above-mentioned series of reactions.

When the protease reaction is included, the protease must be completely removed after reaction because the remaining protease might otherwise have serious harmful influences on the subsequent enzymatic reactions. For this purpose, suspension, centrifugation and removal of a supernatant need to be repeated for washing, leading to loss of the microbeads. Since the protease treatment can be omitted according to the method of the present invention, the operation can be simplified, the rate of loss of microbeads can be reduced, and the efficiencies of the subsequent enzymatic reactions (which may be reduced due to incomplete removal of a protease) can be increased.

The DNA microbead array of the present invention is prepared using the DNA immobilization method of the present invention as described above. Since the DNA microbead array of the present invention is prepared using a highly efficient reaction, more moles of target DNAs are immobilized onto a single microbead as compared with the conventional microbead array. Thus, if the DNA microbead array of the present invention is applied to the method of the Literature 1 for gene expression analysis, sorting can be carried out more accurately because the fluorescence intensity for a single bead after competitive hybridization becomes greater. Furthermore, if the DNA microbead array is applied to the method of the Literature 2 for nucleotide sequence analysis, the S/N ratio and, consequently, the number of nucleotides that can be analyzed can be increased because the fluorescence intensity for a single bead becomes greater. As a result, the number of sequences that can be obtained in one round of analysis can be increased.

Examples

The following examples further illustrate the present invention in detail but are not to be construed to limit the scope thereof.

Referential Example 1: Hybridization of target DNA having single-stranded tag with anti-tag on microbead
Microbeads having anti-tags were prepared according to the method as described in the Literature 1.

Human promyelocytic leukemia cell line HL-60 (ATCC CCL-240) was cultured in RPMI 1640 medium containing 10% fetal bovine serum. Total RNA was then prepared using TRIzol reagent (Gibco) from the cells collected by centrifugation. mRNA was purified from the total RNA using Oligotex™-dT30 (Takara Shuzo). cDNA synthesized using this mRNA as a template according to the method as described in the Literature 1 was inserted into a tag vector. Six plasmid pools each consisting of 140,000 to 180,000 clones were prepared. A PCR was carried out using the plasmid pools as a template, the resulting product was digested with a restriction enzyme PacI (New England Biolabs (NEB)), and the tag portion was converted into single-stranded DNAs using T4 DNA polymerase (NEB) according to the method as described in the Literature 1.

144 million microbeads having anti-tags and 300 μg of cDNAs having single-stranded tags were mixed together and subjected to hybridization in 400 μl of a hybridization buffer (10 mM phosphate buffer (pH 7.2), 0.5 M NaCl, 0.01% Tween 20, 1.2% dextran sulfate) at 72°C for 3 days while shaking. The microbeads were collected by centrifugation, and then suspended and washed in 500 μl of a solution (10 mM Tris-HCl (pH 7.9), 10 mM MgCl$_2$, 50 mM NaCl, 1 mM dithiothreitol (DTT), 0.01% Tween 20) at 64 to 70°C for 30 minutes while shaking. After repeating the washing procedure several more times, the microbeads for the six pools were combined, and microbeads in a given population were isolated therefrom. The population consisted of 1% of the microbeads that were determined to emit the greatest intensities of fluorescence from FAM using MoFlo cytometer (Cytomation). About six million microbeads to which cDNAs were bound as a result of hybridization were obtained by the above-mentioned procedure.

Example 1: Simultaneous reactions using DNA ligase and DNA polymerase

(1) The following reactions were carried out according to the method of Brenner et al. Two million microbeads to which cDNAs were bound as a result of hybridization, which were prepared as described above in Referential Example 1, were suspended in 100 µl of a solution containing 10 mM Tris-HCl (pH 7.9), 10 mM $MgCl_2$, 50 mM NaCl, 1 mM DTT, 0.01% Tween 20, and 0.1 mM each of dATP, dGTP, dTTP and 5-methyl dCTP. 20 U of T4 DNA polymerase was added thereto. The resulting mixture was reacted at 12°C for 30 minutes while shaking.

The microbeads were collected by centrifugation, washed in 500 µl of phosphate buffered saline containing 0.01% Tween 20, and suspended in a Pronase reaction mixture (phosphate buffered saline containing 0.14 mg/ml Pronase and 1 mM $CaCl_2$) for reaction at 37°C for 1 hour while shaking. The mixture was centrifuged, and a supernatant was discarded. The microbeads were suspended in 1 ml of a solution containing 10 mM Tris-HCl buffer (pH 8.0), 1 mM EDTA and 0.01% Tween 20. The procedure comprising centrifugation, removal of a supernatant and suspension was repeated two more times to obtain a suspension of microbeads from which Pronase was removed. The procedure as described in this paragraph is hereinafter referred to as "Pronase treatment."

The microbeads were suspended in 100 µl of a solution containing 10 mM Tris-HCl (pH 7.9), 10 mM $MgCl_2$, 50 mM NaCl, 1 mM DTT, 0.01% Tween 20 and 1 mM ATP. 400 U of T4 DNA ligase (NEB) was added thereto. The mixture was reacted at 37°C for 1 hour while shaking, and then subjected to the Pronase treatment.

Two mixtures were prepared, each of which was prepared by adding 2 µl of the thus obtained microbead suspension to 300 µl of a solution containing 10 mM Tris-HCl buffer (pH 8.0), 1 mM EDTA and 0.01% Tween 20. One of the mixtures was not subjected to heating (Sample A), whereas the other was subjected to a reaction at 72°C for 30 minutes while shaking (Sample B). The distribution of intensities of fluorescence from FAM was determined for the microbeads in the samples using Coulter EPICS XL (Beckman Coulter). The efficiency of covalent bond formation reaction was calculated according to the following equation:

Efficiency of covalent bond formation reaction (%) = (Mean fluorescence intensity for Sample B) / (Mean fluorescence intensity for Sample A) x 100

As a result, the efficiency of covalent bond formation reaction according to the conventional method as described in the Literature 1 was determined to be 42.1%.

(2) Two million microbeads to which cDNAs were bound as a result of hybridization, which were prepared in Referential Example 1, were suspended in 100 µl of a solution containing 10 mM Tris-HCl (pH 7.9), 10 mM $MgCl_2$, 50 mM NaCl 1.4 mM DTT, 0.01% Tween 20, 0.1 mM each of dATP, dGTP, dTTP and 5-methyl dCTP, and 1 mM ATP. 20 U of T4 DNA polymerase and 400 U of T4 DNA ligase were added thereto. The resulting mixture was reacted at 12°C for 2 hours while shaking. The mixture was then subjected to the Pronase treatment (Pronase Treatment 1). The efficiency of covalent bond formation reaction was determined as described in Example 1-(1).

As a result, the efficiency of covalent bond formation reaction was determined to be 88.9%. As compared with the conventional method as described in Example 1-(1), the efficiency of covalent bond formation reaction was increased about twice by reacting a DNA polymerase and a DNA ligase at the same time.

Example 2: Omission of Pronase reaction

(1) The following reactions were carried out according to the method of Brenner et al. The microbeads treated with T4 DNA polymerase and T4 DNA ligase followed by Pronase in Example 1-(2) were collected by centrifugation. The collected microbeads were suspended in 500 µl of a DpnII buffer (50 mM Bis Tris-HCl (pH 6.0), 10 mM $MgCl_2$, 1 mM DTT, 100 mM NaCl, 0.01% Tween 20). The suspension was centrifuged, and a supernatant was removed. The microbeads were suspended in 100 µl of the DpnII buffer. 150 U of DpnII (NEB) was added thereto. The mixture was reacted at 37°C overnight while shaking. The microbeads were then subjected to the Pronase treatment (Pronase Treatment 2).

The microbeads collected by centrifugation were suspended in 500 µl of a solution containing 10 mM Tris-HCl (pH 7.5), 5 mM $MgCl_2$, 7.5 mM DTT and 0.01% Tween 20. The suspension was centrifuged, and a supernatant was removed. The microbeads were suspended in 100 µl of the above-mentioned buffer containing 0.033 mM dGTP. 10 U of the large fragment of DNA polymerase I (NEB) was added thereto. The mixture was reacted at 25°C for 15 minutes while shaking. The microbeads were subjected to the Pronase treatment (Pronase Treatment 3).

Subsequently, the microbeads collected by centrifugation were suspended in 500 µl of a solution containing 10 mM Tris-HCl (pH 7.9), 10 mM $MgCl_2$, 1 mM DTT, 0.01% Tween 20. The suspension was then centrifuged, and a supernatant was removed. The microbeads were suspended in 100 µl of a solution containing 10 mM Tris-HCl (pH 7.9), 10 mM $MgCl_2$, 1 mM DTT, 0.01% Tween 20, 1 mM ATP and 5 µM adaptor. 2000 U of T4 DNA ligase was added thereto. The mixture was reacted at 16°C overnight while shaking. The adaptor used for this reaction was prepared by annealing a synthetic DNA having the sequence of SEQ ID NO:1 to a synthetic DNA having the sequence of SEQ ID NO:2. 6-Carboxyl fluorescein (FAM) was added via two spacers at the 3'-terminus of the synthetic DNA having the sequence of SEQ ID NO:2. The microbeads were subjected to the Pronase treatment (Pronase Treatment 4). The structural formula of the spacer is shown below:

$$-O- (CH_2)_2-O- [(CH_2)_2-O]_4-(CH_2)_2-O-POOH-$$

After the Pronase Treatment 4, 2 µl of the microbead suspension were added to 300 µl of a solution containing 10 mM Tris-HCl (pH 8.0), 1 mM EDTA and 0.01% Tween 20 (Sample C). The distribution of intensities of fluorescence from FAM was determined for the microbeads in the sample using Coulter EPICS XL. The efficiency of adaptor ligation reaction was calculated according to the following equation:

Efficiency of adaptor ligation reaction (%) = (Mean fluorescence intensity for Sample C) / (Mean fluorescence intensity for Sample A in Example 1-(2)) x 100

As a result, the efficiency of adaptor ligation reaction according to the conventional method of Brenner et al. was determined to be 93.1%.

After the Pronase Treatment 4, the microbeads were collected by centrifugation, and suspended in 500 µl of a bead stripping solution (150 mM NaOH, 0.01% Tween 20). The suspension was centrifuged, and a supernatant was removed. The microbeads were resuspended in 500 µl of the bead stripping solution. The suspension was reacted at room temperature for 10 minutes. The suspension was centrifuged, and a supernatant was removed. The microbeads were suspended in 1 ml of a solution containing 10 mM Tris-HCl (pH 8.0), 1 mM EDTA and 0.01% Tween 20. The procedure comprising centrifugation, removal of a supernatant and resuspension was repeated two more times to obtain a microbead suspension.

2 µl of the microbead suspension were added to 300 µl of a solution containing 10 mM Tris-HCl (pH 8.0), 1 mM EDTA and 0.01% Tween 20 (Sample D). The distribution of intensities of fluorescence from FAM was determined for the microbeads in the sample using Coulter EPICS XL. The efficiency of conversion of target DNAs into single-stranded DNAs was calculated according to the following equation:

Efficiency of conversion of target DNAs into single-stranded DNAs (%) = {1 - (Mean fluorescence intensity for Sample D) / (Mean fluorescence intensity for Sample C)} x 100

As a result, the efficiency of conversion of target DNAs into single-stranded DNAs according to the conventional method of Brenner et al. was determined to be 89.0%.

(2) The same procedure as that described in Example 2-(1) was carried out except that the Pronase Treatment 3 was not carried out. As a result, the efficiency of adaptor ligation reaction and the efficiency of conversion of target DNAs into single-stranded DNAs were determined to be 103.6% and 89.2%, respectively. Thus, even if the Pronase Treatment 3 was not carried out, reaction efficiencies equivalent to those of the conventional method of Brenner et al. were accomplished.

A DNA microbead array can be conveniently prepared with high reaction efficiency according to the method of the present invention. By using the DNA microbead array of the present invention which is prepared according to the method of the present invention, the precision of gene expression analysis can be increased, and the number of nucleotides that can be analyzed for a single sequence as well as the number of sequences that can be obtained in one round of sequence analysis can be increased. Sequence Listing Free Text:

SEQ ID NO:1: Designed oligonucleotide for adaptor.

SEQ ID NO:2: Designed oligonucleotide for adaptor.

## Claims

1. A method for immobilizing a DNA upon preparation of a DNA microbead array, the method comprising forming a covalent bond between a DNA to be immobilized and an anti-tag DNA on a microbead using a DNA polymerase and a DNA ligase, wherein the DNA polymerase and the DNA ligase are reacted at the same time.

2. The method according to claim 1, wherein the DNA polymerase substantially has a DNA synthesis activity as well as a 5'→3' exonuclease activity and/or a 3'→5' exonuclease activity.

3. The method according to claim 2, wherein the DNA polymerase is at least one selected from the group consisting of T4 DNA polymerase, Escherichia coli DNA polymerase I, the Klenow fragment of Escherichia coli DNA polymerase I, Taq DNA polymerase and Pfu DNA polymerase.

4. The method according to claim 1, wherein the DNA ligase is at least one selected from the group consisting of T4 DNA ligase, Escherichia coli DNA ligase and Taq DNA ligase.

5. The method according to claim 1, wherein the concentration of the DNA polymerase ranges from 20 to 2,000 U/ml and the concentration of the DNA ligase ranges from 200 to 50,000 U/ml.

6. A DNA microbead array prepared using the method defined by claim 1.


Sequence Listing

<110> TAKARA BIO INC.

<120> Method for immobilizing DNA

<130> 38-48

<150> JP 2001-164846

<151> 2001-05-31

<160> 2

<210> 1

<211> 14

<212> DNA

<213> Artificial Sequence

<220>

<223> Designed oligonucleotide for adaptor

<400> 1

gactggcagc tcgt                                                    14

<210> 2

```
<211> 17

<212> DNA

<213> Artificial Sequence


<220>

<223> Designed oligonucleotide for adaptor


<400> 2

atcacgagct gccagtc                                        17
```